(19) Europäisches Patentamt
European Patent Office
Office européen des brevets

(11) Veröffentlichungsnummer: **0 355 754**
**A2**

(12) # EUROPÄISCHE PATENTANMELDUNG

(21) Anmeldenummer: 89115308.2

(22) Anmeldetag: 19.08.89

(51) Int. Cl.⁴: C07C 43/315 , C07C 47/565 , C25B 3/02

(30) Priorität: 25.08.88 DE 3828792

(43) Veröffentlichungstag der Anmeldung: 28.02.90 Patentblatt 90/09

(84) Benannte Vertragsstaaten: BE CH DE FR GB IT LI NL

(71) Anmelder: BASF Aktiengesellschaft
Carl-Bosch-Strasse 38
D-6700 Ludwigshafen(DE)

(72) Erfinder: Grosse-Brinkhaus, Karl-Heinz, Dr.
Kaiserslauterer Strasse 112
D-6702 Bad Duerkheim(DE)
Erfinder: Köhler, Ulrich, Dr.
Werderstrasse 48
D-6900 Heidelberg(DE)

(54) **Neue 2-Benzyloxibenzaldehyddialkylacetale, ihre Herstellung und Verwendung.**

(57) 2-Benzyloxibenzaldehyddialkylacetale der allgemeine Formel

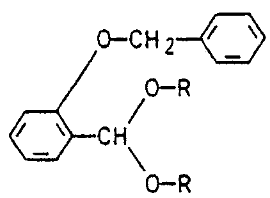

I,

in der R einen Alkylrest mit 1-4 C-Atomen bedeutet oder beide Reste R zusammen für den Alkylenrest $-CH_2-CH_2-$ oder $-CH_2-CH_2-CH_2-$ stehen sowie ihre Herstellung durch elektrochemische Oxidation von 2-Benzyloxitolulol

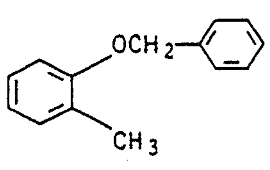

II

in Gegenwart eines Alkanols mit 1 bis 4 C-Atomen oder von Ethylenglykol oder 1,3-Propylenglykol.

EP 0 355 754 A2

# Neue 2-Benzyloxibenzaldehyddialkylacetale, ihre Herstellung und Verwendung

Diese Erfindung betrifft neue 2-Benzyloxibenzaldehyddialkylacetale, ihre Herstellung durch elektrochemische Oxidation von 2-Benzyloxitoluol in Gegenwart von Alkoholen und die Verwendung der neuen Acetale für die Herstellung von 2-Hydroxibenzaldehyd.

Die neuen 2-Benzyloxibenzaldehyddialkylacetale haben die allgemeine Formel

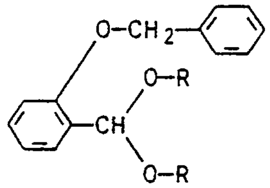

I,

in der R einen Alkylrest mit 1-4 C-Atomen bedeutet oder beide Reste R zusammen für den Alkylenrest $-CH_2-CH_2-$ oder $-CH_2-CH_2-CH_2-$ stehen.

Diese neuen 2-Benzyloxibenzaldehyddialkylacetale sind wertvolle Zwischenprodukte, die man z.B. durch Hydrolyse leicht in 2-Hydroxibenzaldehyd überführen kann. 2-Hydroxibenzaldehyd findet u.a. als Geruchstoff Verwendung und wird z. B. in der Synthese von Cumarin und Chelatbildnern eingesetzt.

Die neuen 2-Benzyloxibenzaldehyddialkylacetale der Formel I lassen sich besonders vorteilhaft dadurch herstellen, daß man 2-Benzyloxitoluol

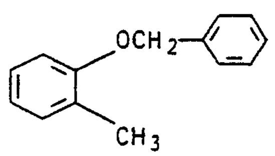

II

in Gegenwart eines Alkanols mit 1 bis 4 C-Atomen oder von Ethylenglykol oder 1,3-Propylenglykol elektrochemisch oxidiert.

Das als Ausgangsstoff benötigte 2-Benzyloxitoluol der Formel II läßt sich auf einfache Weise, z. B. durch Umsetzung von 2-Kresol mit Benzylchlorid, herstellen und kann ohne besondere Reinigung zur Elektrolyse eingesetzt werden.

Für die elektrochemische Oxidation ist keine spezielle Elektrolysezelle erforderlich; sie kann sowohl in geteilten als auch ungeteilten Elektrolysezellen durchgeführt werden. Bevorzugt elektrolysiert man in einer ungeteilten Durchflußzelle. Als Anoden können alle Anodenmaterialien, die unter den Elektrolysebedingungen stabil sind, eingesetzt werden. Bevorzugt verwendet man Graphit. Als Kathodenmaterialien sind u.a. Graphit, Eisen, Stahl, Nickel oder auch Edelmetalle, wie Platin geeignet.

Der bei der Elektrooxidation eingesetzte Elektrolyt hat beispielsweise folgende Zusammensetzung:

1-49 Gew.-% 2-Benzyloxitoluol
0,1-5 Gew.-% Leitsalz
50-95 Gew.-% Alkanol oder Diol

Als Leitsalze kommen die in der organischen Elektrochemie üblichen Leitsalze, wie Salze der Tetrafluoroborsäure, Salze von Alkyl- oder Arylsulfonsäuren oder Salze von Alkylschwefelsäuren, Fluoride, wie KF sowie Alkoholate und Hydroxide, wie $NaOCH_3$ bzw. KOH in Betracht.

Die Stromdichten liegen bei dem erfindungsgemäßen Verfahren z.B. zwischen 0,5 und 15 A/dm$^2$, vorzugsweise bei 1 bis 6 A/dm$^2$. Die Elektrolysetemperaturen sind nach oben hin durch den Siedepunkt des Elektrolyten begrenzt. Zweckmäßigerweise elektrolysiert man bei Temperaturen von Raumtemperatur bis etwa 5 °C unterhalb des Siedepunktes des Elektrolyten.

Die Aufarbeitung der Elektrolyseausträge nimmt man nach an sich bekannten Methoden vor. Zweckmäßigerweise wird der Elektrolyseaustrag destillativ - aufgearbeitet. Überschüssiger Alkohol wird zunächst abdestilliert, das Leitsalz abfiltriert und das rohe 2-Benzyloxibenzaldehyddialkylacetal reindestilliert. Der Alkohol, das Leitsalz und gegebenenfalls auch unumgesetztes 2-Benzyloxitoluol könnnen zur Elektrolyse zurückgeführt werden.

Bei ihrer Verwendung für die Herstellung von 2-Hydroxibenzaldehyd verfährt man z.B. so, daß man die 2-Benzyloxibenzaldehyddialkylacetale auf an sich bekannte Weise, z.B. entsprechend der in der DE-OS 2 904 315 beschriebenen Methode, mit Halogenwasserstoffsäure unter gleichzeitiger Rückgewinnung des Benzylhalogenids umsetzt.

Beispiel 1

Elektrosynthese von 2-Benzyloxibenzaldehyddimethylacetal

Man elektrolysiert 2-Benzyloxitoluol in der wie folgt beschriebenen Elektrolysezelle unter den angegebenen Bedingungen.

Elektrolyseapparatur: ungeteilte Durchflußzelle mit 15 bipolaren Graphitelektroden

Elektrolyt:

111,6 g (10 Gew.-%) 2-Benzyloxitoluol

4,5 g (0,4 Gew.-%) $C_6H_5SO_3Na$
1000 g (89,6 Gew.-%) Methanol
Temperatur: 60 °C
Stromdichte: 3 A/dm²
Durchfluß: 190 l/h
Elektrolyse mit 7 F/mol 2-Benzyloxitoluol

Aufarbeitung:

Nach Beendigung der Elektrolyse wird Methanol bei Normaldruck abdestilliert, das ausgefallene Leitsalz abfiltriert und das Filtrat bei 70 bis 155 °C im Vakuum (0,2 mbar) reindestilliert. Hierbei erhält man 4,2 g (3,8 %) unumgesetztes 2-Benzyloxitoluol, 2,5 g (1,9 %) 2-Benzyloxibenzylmethylether und 75,7 g (52 %) 2-Benzyloxibenzaldehyddimethylacetal.
Sdp.: 148 °C/0,2 mbar
MS: m/e = 258
$^1$H-NMR ($CDCl_3$, 300 MHz):
δ = 7,5-7,6 (m, 1H); 7,2-7,45 (m, 6H); 6,9-7,05 (m, 2H); 5,73 (s, 1H); 5,12 (s, 2H); 3,38 (s, 6H) ppm.

Beispiel 2

Elektrosynthese von 2-Benzyloxibenzaldehyddiethylacetal

Man elektrolysiert 2-Benzyloxitoluol in der in Beispiel 1 beschriebenen Elektrolysezelle unter den wie folgt angegebenen Bedingungen.

Elektrolyt:

55,4 g (5 Gew.-%) 2-Benzyloxitoluol
3,5 g (0,3 Gew.-%) $C_6H_5SO_3Na$
1050 g (94,7 Gew.-%) Ethanol
Temperatur: 60 °C
Stromdichte: 1 A/dm²
Durchfluß: 190 l/h
Elektrolyse mit 10,1 F/mol 2-Benzyloxitoluol

Aufarbeitung analog Beispiel 1.

Nach fraktionierter Destillation erhält man 1,6 g (2,8 %) unumgesetztes 2-Benzyloxitoluol, 1,2 g (1,8 %), 2-Benzyloxibenzylethylether und 19,2 g (23, %) 2-Benzyloxibenzaldehyddiethylacetal.
Sdp.: 181 °C/0,8 mbar
MS: m/e = 286
$^1$H-NMR ($CDCl_3$, 200 MHz):
δ = 7,5-7,65 (m, 1H); 7,2-7,5 (m, 6H); 6,9-7,1 (m, 2H); 5,85 (s, 1H); 5,13 (s, 2H); 3,45-3,8 (m, 4H); 1,23 (t, 6H) ppm.

Beispiel 3

Elektrosynthese von 2-(2'-Benzyloxiphenyl)-1,3-dioxolan

Man elektrolysiert 2-Benzyloxitoluol in der in Beispiel 1 beschriebenen Apparatur unter den wie folgt angegebenen Bedingungen.

Elektrolyt:

176,3 g (12,8 Gew.-%) 2-Benzyloxitoluol
4,5 g (0,3 Gew.-%) $C_6H_5SO_3Na$
900 g (65,2 Gew.-%) Methanol
300 g (21,7 Gew.-%) Ethylenglykol
Temperatur: 60 °C
Stromdichte: 3 A/dm²
Durchfluß: 190 l/h
Elektrolyse mit 12,5 F/mol 2-Benzyloxitoluol

Aufarbeitung analog Beispiel 1.

Nach Destillation erhält man 5 g unumgesetztes 2-Benzyloxitoluol, 97,6g 2-Benzyloxibenzaldehyddimethylacetal und 23,3 g 2-(2'-Benzyloxiphenyl)-1,3-dioxolan.
Sdp.: 183 °C/0,5 mbar
MS: m/e = 256
$^1$H-NMR ($CDCl_3$, 300 MHz):
δ = 7,5-7,6 (m, 1H); 7,2-7,5 (m, 6H); 6,9-7,05 (m, 2H); 6,25 (s, 1H); 5,14 (s, 2H); 4,0-4,2 (m, 2H) ppm.

**Ansprüche**

1. 2-Benzyloxibenzaldehyddialkylacetale der allgemeinen Formel

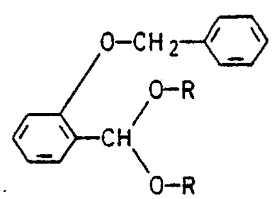

I,

in der R einen Alkylrest mit 1-4 C-Atomen bedeutet oder beide Reste R zusammen für den Alkylenrest $-CH_2-CH_2-$ oder $-CH_2-CH_2-CH_2-$ stehen.

2. Verfahren zur Herstellung von 2-Benzyloxibenzaldehyddialkylacetalen nach Anspruch 1, dadurch gekennzeichnet, daß man 2-Benzyloxitoluol

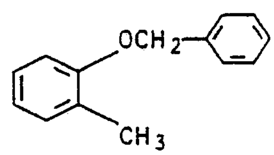

II

in Gegenwart eines Alkanols mit 1 bis 4 C-Atomen oder von Ethylenglykol oder 1,3-Propylenglykol

elektrochemisch oxidiert.

3. Verwendung der 2-Benzyloxibenzaldehyddialkylacetale nach Anspruch 1 für die Herstellung von 2-Hydroxibenzaldehyd.